# EUROPEAN PATENT SPECIFICATION

(11) **EP 3 750 506 B1**
(45) Date of publication and mention of the grant of the patent: **07.06.2023**
(21) Application number: 20179857.6
(22) Date of filing: 12.06.2020
(51) Int. Cl.: A61C 13/00, A61C 1/08

(54) **DENTURE SYSTEM AND METHOD FOR MANUFACTURING A DENTURE**
ZAHNPROTHESESYSTEM UND VERFAHREN ZU DESSEN HERSTELLUNG
PROTHÈSE DENTAIRE ET SA METHODE DE FABRICATION

(30) Priority: 12.06.2019 BE 201905380
(43) Date of publication of application: 16.12.2020
(73) Proprietor: Dental Vision B.V.B.A., 3080 Tervuren (BE)
(72) Inventor: DE CLERCK, René, 3080 Tervuren (BE)
(74) Representative: Callewaert, Koen

(56) References cited:
- BE-A3- 1 020 773
- IT-A1- TO20 110 894
- US-A1- 2011 033 820

## Description

The invention generally concerns a dental prosthesis that is mounted on implants in a patient's oral cavity. More specifically, the invention includes a dental prosthesis system in which a drilling template is designed and manufactured to insert implants in the jaw, as well as a dental prosthesis to be mounted on these implants.

Such systems are for example disclosed in US2011/033820 and ITTO20110894.

Thus, the invention concerns a method for manufacturing a drilling template and a support piece for positioning this drilling template in relation to a jaw's bone. This drilling template is provided with at least one guide hole for drilling into the jaw's bone in view of the insertion of implants for fitting a dental prosthesis. According to this method, a digital mould of the jaw is generated in such a way that this mould can connect virtually perfectly to soft tissue of the jaw. Based on this digital mould, a digital model for a dental prosthesis will be designed that connects in an unambiguous way to the soft tissue of the jaw.

Currently, an attempt is made to place the permanent dental prosthesis on one or more implants in the bone of the jaw immediately after they have been inserted, without the patient being required to respect an ingrowth period for the implants. In order to allow such an immediate strain, it is required that the implants are inserted in the jaw with very high precision in a pre-planned position and with a predetermined orientation. In addition, it is also required to design and manufacture the dental prosthesis with very high accuracy so that it can be fittingly mounted on the implants.

Such a carefully planned insertion of implants in the jaw also allows, at a later date, for example, when a prosthesis mounted on these implants needs to be replaced by a new dental prosthesis, to use the initial data relating to the planning and insertion of the implants to manufacture a new prosthesis that can be immediately attached to the existing implants. In addition, it is possible to provide additional implants if necessary, taking into account the accurately known position of the implants that are already present.

For this purpose, a provisional removable dental prosthesis is manufactured on the basis of the aforementioned digital model of the dental prosthesis, wherein this provisional dental prosthesis is positioned on the relevant jaw and at least one digital image of the jaw with the provisional removable dental prosthesis is generated by tomography. Such an image is preferably a 3D image and is thus, for example, obtained by performing a CT scan. Next, a position and orientation for at least one implant are selected based on this image.

On the basis of the digital model of the dental prosthesis, a digital model of a bone crest part is generated, wherein this bone crest part must extend opposite the crest of the jaw and contains prosthetic teeth whose shape and mutual position correspond to prosthetic teeth provided on said dental prosthesis. Furthermore, based on the digital model of the dental prosthesis, a digital model is also generated for the aforementioned support piece. This support piece should extend at least partially opposite the bone of the jaw which, preferably, implies that the support piece extends at least partially between the jaw and the opposite lip or cheek. More specifically, the support piece extends vestibularly and/or palatally opposite the bone of an upper jaw or vestibularly and/or lingually opposite the bone of a lower jaw, depending on the anatomy of the jaw in question. Coupling devices are hereby provided on the support piece to detachably connect the bone crest part to the support piece. The bone crest part and support piece thus designed are manufactured on the basis of the digital models thereof.

On the basis of the position and orientation selected for said at least one implant, the drilling template is then designed in such a way that it can be attached to the support piece.

Practically, one chooses a direction of entry for said at least one implant based on said X-ray image, designing said digital model for said support piece in such a way that it leaves this direction of entry free for a drilling in accordance with this direction of entry and for inserting said implant in the jaw when said support piece is positioned fittingly on the jaw.

Interestingly, during the manufacture of said provisional prosthesis, at least one reference element, which is clearly visible in said image, is firmly connected to the prosthesis.

Advantageously, said digital mould of said jaw is generated by scanning and digitizing a physical mould of the jaw, which physical mould connects virtually fittingly to soft tissue of the jaw. For said physical mould, an already available removable denture is used, for example.

According to a preferred embodiment of the method according to the invention, fastening means are provided on the support piece to allow it to be attached to the bone of the jaw.

In an interesting way, said physical mould of the jaw is scanned by performing an optical scan, in particular a laser scan, of at least the side of the mould facing the soft tissue of the jaw.

According to an interesting embodiment of the method according to the invention, the aforementioned image generated by tomography includes a three-dimensional X-ray image of the jaw with said bone crest part and said support piece. This three-dimensional X-ray image is, for example, a CT scan.

The invention also concerns a dental prosthesis system,as defined in the claims, with a provisional removable dental prosthesis provided with prosthetic teeth for a lower or upper jaw, wherein this provisional dental prosthesis can be placed fittingly on the lower or upper jaw, wherein the dental prosthesis system includes a support piece and a bone crest part with coupling devices for connecting the support piece and the bone crest part in a detachable manner to each other. The bone crest part should extend opposite the bone crest of the relevant jaw and contains prosthetic teeth corresponding to those of the provisional dental prosthesis, while the support piece extends at least partially, preferably vestibularly, opposite the bone of this jaw with a recess being provided in the support piece which should extend opposite the bone crest. The presence of this recess makes it possible to secure at least one implant in the jaw through the recess. In general, the support piece should extend vestibularly and/or palatally to the bone of an upper jaw or vestibularly and/or lingually to the bone of a lower jaw, depending on the anatomy of the relevant jaw.

The dental prosthesis system according to the invention is characterized in that the whole of the support piece and the bone crest part, when attached to each other by means of said coupling devices, can be fittingly placed in the same position on the jaw as said provisional dental prosthesis.

The dental prosthesis system according to the invention comprises a drilling template that can be attached to said support piece in order to allow the drilling template to be positioned in relation to said bone crest when the support piece is fittingly placed on the jaw. The drilling template has at least one opening for guiding a drill to make a bore hole in the bone of said jaw and to insert an implant in the bore hole through this opening.

Other peculiarities and advantages of the method and the dental prosthesis system according to the invention will become apparent from the following description of a few particular embodiments of the invention; this description is given as an example only and does not limit the scope of the claimed protection in any way; the reference numbers used hereinafter refer to the attached figures.
Figure 1 is a schematic perspective view from a lingual direction of a model for a dental prosthesis for a lower jaw.
Figure 2 is a schematic perspective view from the buccal side of the model in figure 1.
Figure 3 is a schematic perspective view from the lingual direction of the model for a dental prosthesis that is divided into a bone crest part and a support piece according to the invention.
Figure 4 is a schematic perspective view from the buccal side of the model of a dental prosthesis from figure 3, which is divided into a bone crest part and a support piece according to the invention.
Figures 5 and 6 are schematic perspective views of the support piece according to the invention from figures 3 and 4.
Figures 7 and 8 are schematic perspective views of the bone crest part according to the invention from figures 3 and 4.
Figure 9 is a schematic perspective view of the support piece and the bone crest part from figures 5 to 8 when they are detachably connected to each other.
Figure 10 is a schematic perspective view of the support piece from the previous figures to which a drilling template is attached.
Figure 11 is a schematic perspective view of the support piece from the previous figures wherein it includes fastening means that allow it to be attached in a fixed position to the bone of the jaw.

In the different figures, the same reference numbers refer to the same or analogous elements.

In general, the invention concerns a method and a dental prosthesis system that allows implants to be inserted with very high accuracy in a patient's jaw according to a planned position and orientation. More specifically, the invention allows implants to be inserted with a tolerance of less than 35 µm. As a result of this high accuracy in implant insertion, it is therefore possible to manufacture a dental prosthesis based on the planned position of the implants. This dental prosthesis can then be mounted on these implants immediately after the implants have been inserted in the jaw.

This not only offers the advantage that the preparations for manufacturing and placing a permanent dental prosthesis can be done in a shorter period of time and with fewer intermediate steps, but in addition the patient also has a permanently finished dental prosthesis immediately after the implants have been inserted.

The invention is interesting for placing implants with a dental prosthesis in an edentulous jaw, but it can also be applied to a jaw on which a number of teeth are still present.

It goes without saying that the invention can be applied to both the lower jaw and the upper jaw of a person.

Thus, in general, according to the invention, a dental prosthesis is designed. Starting from this design for the dental prosthesis, a support piece 3 and a bone crest part 2 are manufactured which can be detachably connected to each other in order to obtain a whole whose external shape corresponds to that of the designed dental prosthesis. This whole is placed on the corresponding jaw and positioned on the latter. Subsequently, the support piece 3 is fixed in relation to the jaw, and the bone crest part 2 is removed from it so that a drilling template 9 designed for this purpose can be attached to the support piece 3. The required implants are then inserted into the jaw using the drilling template 9 according to a planned position and orientation. The support piece 3 and the drilling template 9 are then removed from the jaw in order to attach the dental prosthesis to the implants.

According to an interesting embodiment of the method according to the invention, a physical mould, in particular an anatomical mould, is made of the jaw on which a dental prosthesis carried by implants is to be inserted. Such a mould is made for example of alginate in a manner known as such. A mould is made of the oral tissue, in particular of soft tissue of the jaw, preferably including the transition from jaw to cheek, the so-called oral fold.

The physical mould of the jaw obtained in this way therefore virtually fits to the soft tissue of the jaw. The side of the mould connecting to the jaw is scanned in order to digitize it and thus generate a digital mould of the jaw. This involves digitising the surface of the jaw, which is normally formed mainly by soft tissue, more specifically gingiva and mucosa, to obtain a digital mould of the surface of the jaw.

According to an interesting variant of this embodiment of the invention, an existing removable dental prosthesis is used for said physical mould of the jaw.

Scanning the physical mould in order to digitize it and thus obtain a digital mould can be done, for example, by optically scanning the physical mould, such as by performing a three-dimensional laser scan.

Starting from this digital mould, a digital model for a dental prosthesis is designed that can be connected in an unambiguous manner to the soft tissue of the jaw. The design of such a digital model for a dental prosthesis is known as such and is often used, for example, to manufacture a prosthesis using an additive manufacturing technique or by milling.

Normally, when digitally designing a dental prosthesis, a digital simulation of the entire set of teeth is generated in such a way that the mutual position of the teeth in the lower and upper jaw can be selected as optimally as possible.

If a patient already has removable dentures, it is often possible to scan and digitize the surface of these dentures resting on the oral tissue. Thus, these removable dentures are used as a physical mould of the jaw in the method according to the invention, as already mentioned above.

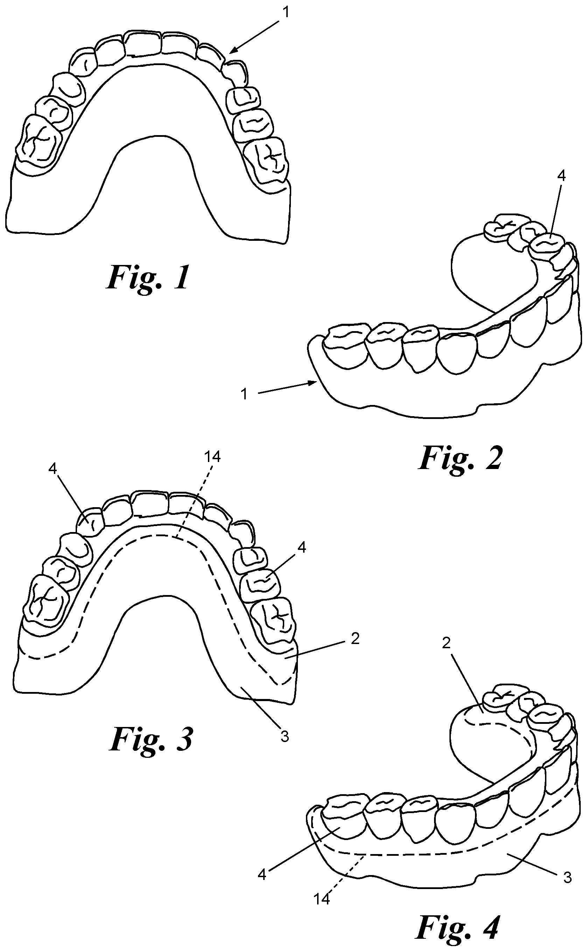

Figures 1 and 2 schematically represent a digital model for a dental prosthesis 1 for a mandible that was designed from a scanned physical mould of the mandible. This digital model is therefore designed in such a way that the side which is to face the jaw can be connected virtually fitting and in an unambiguous way to the soft tissue of this jaw. By soft tissue is meant the gums surrounding the bone of the jaw in question, in particular mucosa and/or gingiva.

Subsequently, a provisional dental prosthesis 1 is manufactured based on said digital model for the dental prosthesis. This dental prosthesis 1 is removable and is manufactured, for example, by milling it out of plastic or by another rapid prototyping technique such as, for example, stereo lithography or selective laser melting or sintering.

This provisional dental prosthesis 1 is then positioned in the oral cavity on the jaw, taking into account, for example, the anatomical structure of the opposite jaw and teeth. More specifically, the provisional dental prosthesis is positioned on the respective jaw in such a way that the prosthetic teeth of the provisional dental prosthesis are correctly positioned in relation to the teeth of the opposite jaw.

After the provisional dental prosthesis 1 has thus been optimally positioned on the relevant jaw in the oral cavity, at least an image of the provisional dental prosthesis, together with the jaw on which it is placed, is generated by means of tomography. Such an image is, for example, an X-ray image and, preferably, a three-dimensional X-ray image or, more specifically, a three-dimensional image obtained by performing a CT scan. This image thus represents the constitution and structure of the bone of the jaw and, in particular, the location of nerve bundles and blood vessels. Normally, this image is digital, such that it can be edited using a computer and displayed on a screen.

Preferably, the provisional dental prosthesis 1 should contain reference elements that are clearly and accurately visible in said image. The use of such reference elements is in itself known to the professional. These reference elements consist, for example, of spherical glass beads attached to the provisional dental prosthesis in a fixed position or embedded therein. The use of such reference elements is particularly useful when the provisional dental prosthesis is not sharply depicted in said image.

Thus, the position of the provisional dental prosthesis 1 in relation to the jaw and the structure of the jaw are defined through these reference elements.

Starting from the thus generated image of the jaw together with the provisional dental prosthesis 1, a position and orientation for one or more implants are then selected for mounting a permanent dental prosthesis. More specifically, position and orientation, together with an insertion direction, are chosen for at least one implant to be attached to the jaw in order to attach the permanent dental prosthesis thereto. Critical structures present in the bone of the jaw, such as blood vessels or nerve bundles, as well as the structure of the bone of the jaw itself, are taken into account, for example, when selecting the position and orientation for the implants.

Advantageously, the digital model generated for the dental prosthesis is digitally merged with the image made of the jaw with the provisional dental prosthesis, such that the model for the dental prosthesis coincides with the image made of the provisional dental prosthesis. This ensures that the selected position and orientation of the implants in the jaw are defined with respect to the model of the dental prosthesis.

Furthermore, in the method according to the invention, the digital model of the dental prosthesis 1 is divided into a digital model for a bone crest part 2 which should extend opposite the crest of the jaw and a digital model for a support piece 3. This support piece 3 preferably stretches at least partially vestibularly to the bone of the jaw. In general, the support piece extends vestibularly and/or palatally to the bone of an upper jaw or vestibularly and/or lingually to the bone of a lower jaw, depending on the anatomy of the jaw. More specifically, the shape of the part of the support piece 3 which extends opposite the bone between the jaw and the opposite cheek or between the jaw and the opposite lip is chosen such that this support piece 3 is sufficiently close to the jaw and is able to support itself against it in order to assume a stable position on the jaw. Figures 3 and 4 show the model of the dental prosthesis 1 with the bone crest part 2 and the support piece 3. A dashed line 14 indicates the separation between the bone crest part 2 and the support piece 3.

The bone crest part 2 should extend opposite the bone crest of the relevant jaw. More specifically, this bone crest part 2 extends opposite the zone of the jaw where possible implants should be placed for mounting the dental prosthesis. As a result, this bone crest part 2 also contains prosthetic teeth 4 corresponding to the model of the dental prosthesis 1.

The part of the digital model of the dental prosthesis 1 which connects to the bone crest part 2 forms said support piece 3. This support piece, as already mentioned above, should extend at least partially vestibularly and/or palatally to the bone of the jaw when it is to be placed on an upper jaw, while the support piece 3 should extend at least partially vestibularly and/or lingually in case of a lower jaw. Furthermore, this support piece 3 has a recess 5 which is provided so as to extend opposite the bone crest of the jaw. When this support piece 3 is thus placed in a fitting manner on the relevant jaw, the recess 5 ensures that the zone of the jaw where possible implants are to be inserted remains free. Preferably, the support piece 3 thus encloses the sides of the relevant jaw which are opposite in relation to the bone crest and thereby connects to the soft tissue of these sides of the jaw in a fitting manner.

In particular, the selected position and orientation for an implant determines a direction of insertion corresponding to the axis direction of the implant after it has been inserted in the jaw. Thus, for each implant, a direction of insertion is also selected based on said image. The digital model for the support piece 3 is therefore designed in such a way that this direction of insertion is free, so that a hole can be made in the jaw in accordance with this direction of insertion when the support piece is positioned in a fitting manner on the jaw and so that said implant can be inserted in this hole. More specifically, this means that said recess 5 ensures that the direction of insertion remains free for each of the selected implants to be inserted in the jaw.

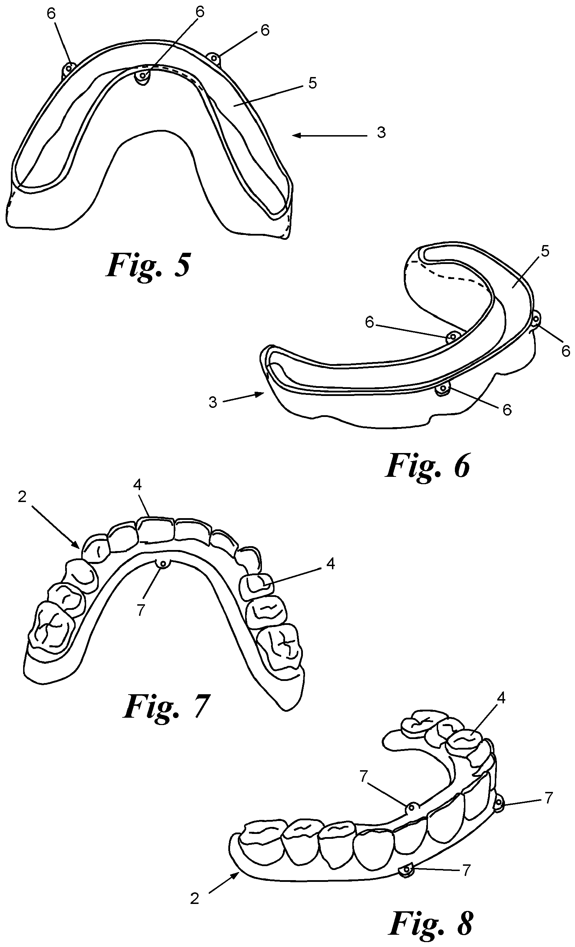

Furthermore, the digital model of the bone crest part 2 and of the support piece 3 is provided with coupling devices that allow the bone crest part 2 to be detachably connected to the support piece 3. These coupling devices allow, for example, to connect the bone crest part and the support piece in an unambiguous manner and/or in a detachable manner. By way of example, figures 5 to 8 show coupling devices formed by several flanges 6 provided with a screw hole on the support piece 3 and with flanges 7 cooperating with the latter which are provided on the bone crest part 2. In this way, the bone crest part 2 and the support piece 3 can be mounted against each other in a fitting manner by placing the respective flanges 6 and 7 against each other and by fixing them together by means of a screw.

It goes without saying that alternative coupling devices can be provided on the bone crest part 2 and/or the support piece 3. For example, the support piece 3 may have a raised edge which extends along the dashed line 14 in such a way that the bone crest part 2 can be placed on the support piece 3 wherein the circumference of the bone crest part 2 fits snugly within this raised edge.

According to yet another embodiment of the invention, the coupling devices are formed, for example, by one or more geometrical elements provided in elevated relief on the side of the bone crest part 2 which is to connect to the support piece 3, wherein these elements can be fittingly inserted into one or more corresponding recesses on the opposite side of the support piece 3 in order to mount the bone crest part and the support piece in a fitting manner against one another. It goes without saying that the raised elements can also be provided on the support piece 3, while corresponding recesses are present in the bone crest part 2. The presence of such geometrical elements and corresponding recesses thus allows the bone crest part and the support piece to be connected to each other in an unambiguously detachable manner. These geometrical elements are for example formed by a cube, a cylinder, a beam-shaped element, etc.

The bone crest part 2 and support piece 3 designed in this way are preferably manufactured by applying a rapid-prototype technique based on their digital models. More specifically, the bone crest part 2 and the support piece 3 are manufactured in plastic by milling or by an additive manufacturing technique such as, for example, stereo lithography. Figures 5 to 8 also show the produced bone crest part 2 and support piece 3.

A drilling template 9 is also digitally designed. This template 9 makes it possible to drill holes in the jaw and to insert the corresponding implants when the drilling template 9 is attached to the support piece 3. To this end, the drilling template 9 has one or more openings 10 for guiding a drill to make a bore hole in the bone of the jaw according to the preselected position and orientation and for placing an implant in this bore hole through said opening 10. Said openings 10 thus extend over the full height of the drilling template 9 and are preferably formed by a cylindrical recess that may have a smooth wall. If necessary, guide sleeves for a drill may be placed in these openings 10, as is known to a person skilled in the art.

This drilling template 9 is thus designed based on the selected position and orientation of the implants, in such a way that it can be attached to the support piece 3. For this purpose, flanges 11 are provided on the template 9, for example, which work in conjunction with flanges 6 of the support piece 3 and thus allow the drilling template 9 to be attached to the support piece 3 in an unambiguous manner.

In general, coupling devices are provided on the drilling template 9 which cooperate with coupling devices of the support piece 3 in order to allow the drilling template to be attached in a detachable manner and in a planned position to the support piece 3. These coupling devices of the drilling template 9 are, for example, similar or identical to those provided on the bone crest part.

The drilling template 9 is designed such that, when the drilling template 9 is attached to the support piece 3, while the support piece 3 is placed on the jaw in a fitting manner, the drilling template 9 allows holes to be drilled in the jaw through said openings 10 and implants to be inserted in this jaw according to the selected position and orientation. When the drilling template 9 is mounted on the support piece 3, it is therefore possible to position the drilling template 9 opposite the bone crest with said openings 10 extending opposite the recess 5 in the support piece 3.

Such a drilling template 9 is then manufactured, for example, in plastic by a rapid-prototype technique, in particular by milling.

Furthermore, the permanent dental prosthesis is designed and manufactured. For this purpose, mounting devices are added to the digital model for the dental prosthesis 1, which allows the dental prosthesis to be mounted on the implants in a pre-planned manner. The selected position and orientation of these implants in the jaw are thereby taken into account. The permanent dental prosthesis is then, preferably, also manufactured by a rapid prototyping technique such as, for example, the additive manufacturing of the prosthesis or milling. The shape of this permanent dental prosthesis then practically corresponds to the shape of the provisional dental prosthesis, the permanent dental prosthesis having additional mounting devices to be fixed to said implants.

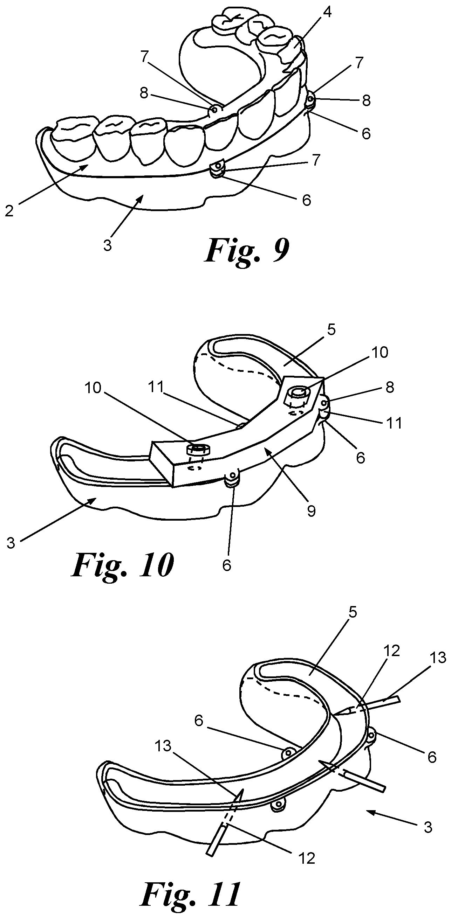

Before proceeding to drilling bore holes in the jaw for inserting the implants, the bone crest part 2 is detachably attached to the support piece 3, as shown schematically in figure 9. The bone crest part 2 and the support piece 3 are attached to each other in a fitting manner, with the flanges 6 and 7 connecting and being secured to each other with a screw 8.

When the bone crest part 2 and the support piece 3 are fitted against one another, they form a whole whose shape corresponds to that of the provisional dental prosthesis 1. Thus, the external shape of the whole of the bone crest part 2 connected to the support piece 3 is almost identical to the external shape of said dental prosthesis. Preferably, this external shape of said whole is almost identical to that of the dental prosthesis, with the exception, for example, of the coupling devices, mounting devices and/or fastening means present.

The bone crest part 2 is thus provided to rest on the jaw by means of the support piece 3, wherein the support piece can be fittingly and unambiguously connected to the relevant jaw, in particular to soft tissue of this jaw.

The whole of the bone crest part 2 and the support piece 3 are placed in the oral cavity on the jaw, ensuring that the whole is correctly positioned in relation to the jaw and that at least the support piece 3 fittingly connects to the soft tissue of the jaw. In particular, it is ensured that this whole formed of the bone crest part 2 and the support piece 3 occupies the same position in relation to the relevant jaw as the position of the provisional dental prosthesis when generating said image.

It is then ensured that the position of the support piece 3 in relation to the jaw is fixed. The bone crest part 2 is then removed from the support piece 3 and the drilling template 9 is attached to the support piece 3 in a detachable way, while the latter retains its position in relation to the jaw. Figure 10 shows the drilling template 9 when it is attached to the support piece 3.

According to an interesting embodiment of the invention, the support piece 3 contains fastening means that allow it to be attached to the bone of the jaw.

Figure 11 shows an example of these fastening means, where they are formed by recesses 12 extending through the support piece 3 between the side facing the bone and the vestibular side. These recesses 12 work in conjunction with needle-shaped anchoring elements 13 which must be fastened through these recesses 12 in the bone of the jaw. In this way, these fastening means allow the support piece 3 to be fixed in relation to the jaw.

After the support piece 3 is fixed in relation to the jaw, the bone crest part 2 is removed and the drilling template 9 is mounted on the support piece 3 so that this drilling template jig 9 takes a fixed and pre-planned position in relation to the jaw.

Subsequently, in a way that is known to those skilled in the art, a bore hole is made in the jaw, according to the direction of insertion, by means of a drill guided through a guide opening 10 of the drilling template 9. An implant is then screwed into the provided bore hole according to said direction of insertion, while also being guided through the corresponding opening 10.

After the implants have thus been inserted in the jaw according to the predetermined position and orientation, the support piece 3, together with the drilling template 9, is removed from the jaw and the permanent dental prosthesis is mounted on the implants using the mounting means. These mounting means include, for example, screw openings that can be connected in a fitting manner to the inserted implants in order to attach the dental prosthesis to the implants using screws.

The drilling template 9 according to the invention preferably has means for placing the implants concerned at a predetermined depth in the bone of the jaw. Such means are described, for example, in document WO 2008/009080 A1 and can also be used in the current invention.

It is possible, for example, that the drilling template 9 is not only attached to the support piece 3, but that it also rests, for example, on teeth that are still present in the jaw.

Furthermore, in certain cases it is also possible to provide the above-mentioned mounting means as of the design stage of the dental prosthesis. The orientation and position of the implants are then selected taking into account the presence of the mounting means. In this case, the provisional prosthesis can also be used as a permanent dental prosthesis and be attached to the inserted implants.

## Claims

1. Method for manufacturing a drilling template (9) and a support piece (3) for positioning this drilling template (9) in relation to the bone of a jaw, the drilling template (9) being provided with at least one guide opening (10) for drilling bore holes in said bone for inserting at least one implant for mounting a dental prosthesis (1), wherein, according to the method, a digital mould of the jaw is generated such that it can be connected virtually fittingly to soft tissue of the jaw, wherein, based on this digital mould, a digital model for a dental prosthesis (1) is designed, such that it can connect to said soft tissue of the jaw, wherein a provisional dental prosthesis is manufactured on the basis of said digital model of the dental prosthesis and this provisional dental prosthesis is positioned on the relevant jaw and at least one image of the jaw with the provisional dental prosthesis (1) is generated by means of tomography, wherein a position and orientation for said at least one implant are then selected on the basis of said image,
**characterised in that,** on the basis of the digital model of the dental prosthesis (1), a digital model for a bone crest part (2) is generated, in which this bone crest part must extend opposite the crest of the jaw and contains prosthetic teeth whose shape and mutual position correspond to prosthetic teeth provided on said dental prosthesis, and wherein, on the basis of the digital model of the dental prosthesis, a digital model for said support piece (3) is also generated, which support piece should extend at least partially opposite the bone of the jaw, wherein coupling devices (6) are provided to the support piece (3) in order to connect the bone crest part (2) to the support piece (3) in a detachable manner, so that the external shape of the whole of the bone crest part connected to the support piece is substantially identical to that of said dental prosthesis,
wherein the thus designed bone crest part (2) and support piece (3) are manufactured on the basis of said digital models, wherein, based on the selected position and orientation of said at least one implant, said drilling template (9) is designed such that this drilling template can be attached to said support piece (3).

2. Method according to claim 1, wherein a direction of insertion for said at least one implant is selected starting from said image, wherein said digital model for said support piece (3) is designed such that this direction of insertion is left clear for drilling according to this direction of insertion and to insert said implant in the jaw when this support piece is positioned in a fitting manner on the jaw.

3. Method according to claim 1 or 2, wherein said digital mould of said jaw is generated by scanning and digitizing a physical mould of the jaw, which physical mould connects in a virtually fitting manner to soft tissue of the jaw.

4. Method according to claim 3, wherein said physical mould of the jaw is scanned by performing an optical scan, in particular a laser scan, of at least part of its side directed towards the soft tissue of the jaw.

5. Method according to any one of claims 3 or 4, wherein for said physical mould an already available removable denture is used.

6. Method according to any one of claims 1 to 5, wherein said digital model for the support piece (3) is designed, as a function of the anatomy of the jaw concerned, such that the support piece (3) extends at least partially vestibularly and/or palatally to the bone of the jaw if it is an upper jaw, whereas if the aforementioned jaw is a lower jaw, this digital model for the support piece (3) is designed such that the support piece (3) can extend vestibularly and/or lingually to the bone of a lower jaw.

7. Method according to any one of claims 1 to 6, wherein mounting means are provided to said digital model of the dental prosthesis so as to allow it to be mounted on said implant after the implant has been fixed in the jaw.

8. Method according to any one of claims 1 to 7, wherein fastening means (12,13) are provided to the support piece (3) so as to allow it to be fixed to the bone of the jaw.

9. Method according to any one of claims 1 to 8, wherein said image generated by means of tomography includes a three-dimensional X-ray image of the jaw with said bone crest part (2) and said support piece (3).

10. Method according to any one of claims 1 to 9, wherein said bone crest part (2) and said support piece (3) are made of plastic by applying a rapid-prototype technique, in particular by milling.

11. Method according to any one of claims 1 to 10, wherein said drilling template (9) is digitally designed, wherein it is made of plastic by means of a rapid-prototype technique, in particular by milling.

12. Dental prosthesis system with a provisional dental prosthesis (1) with prosthetic teeth for a lower or upper jaw, wherein this provisional dental prosthesis can be fittingly placed on the lower or upper jaw, wherein it fits against the soft tissue of the jaw and wherein the dental prosthesis system includes a support piece (3) and a bone crest part (2) with coupling devices (6,7,8) to detachably connect the support piece (3) and the bone crest part (2) to each other, wherein the bone crest part (2) should extend opposite the bone crest of the respective jaw and contains prosthetic teeth corresponding to those of the provisional dental prosthesis, while the support piece (3) should extend at least partially opposite the bone of this jaw and can be placed in a fitting manner on this jaw, wherein a recess (5) is provided in the support piece (3) which should extend opposite the bone crest to allow the attachment of at least one implant in the jaw through this recess, wherein the whole of the support piece (3) and the bone crest part (2), when secured to each other by means of said coupling devices, can be placed in a fitting way on the jaw by means of the support piece (3) in the same position as said provisional dental prosthesis, wherein the external shape of the whole of the bone crest part connected to the support piece (3) is almost identical to that of said dental prosthesis, **characterised in that** it comprises a drilling template (9) which can be detachably fastened to the support piece (3) in order to allow this drilling template (9) to be positioned opposite said bone crest when the support piece (3) is placed on the jaw in a fitting manner, wherein the drilling template (9) has at least one opening (10) for guiding a drill to make a bore hole in the bone of said jaw and to insert an implant in this bore hole through said opening (10).

13. Dental prosthesis system according to claim 12, wherein said support piece (3) is adapted for stretching at least partially vestibularly and/or palatally to the bone of the jaw when the jaw is an upper jaw, whereas when said jaw is a lower jaw, the support piece (3) can stretch vestibularly and/or lingually to the bone of the lower jaw.

14. Dental prosthesis system according to claim 12 or 13, wherein said support piece (3) comprises fastening means (12,13) that allow it to be fixed to the jaw's bone.

15. Dental prosthesis system according to claim 14, wherein said fastening means are formed by recesses (12) extending through the support piece (3) between the side facing the bone and the vestibular side, the palatal side and/or the lingual side of the support piece (3), wherein these recesses (12) cooperate with needle-shaped anchoring elements (13) which must be fastened through these recesses (12) in the jaw's bone.

## Patentansprüche

1. Verfahren zur Herstellung einer Bohrschablone (9) und eines Trägerstücks (3) zur Positionierung dieser Bohrschablone (9) in Bezug auf den Knochen eines Kiefers, wobei die Bohrschablone (9) mit wenigstens einer Führungsöffnung (10) zum Bohren von Bohrlöchern in dem Knochen zum Einsetzen wenigstens eines Implantats zum Montieren einer Zahnprothese (1) versehen ist, wobei gemäß dem Verfahren eine digitale Form des Kiefers erzeugt wird, sodass sie virtuell passend mit Weichgewebe des Kiefers verbunden werden kann, wobei basierend auf dieser digitalen Form ein digitales Modell für eine Zahnprothese (1) gestaltet wird, sodass es mit Weichgewebe des Kiefers verbunden werden kann, wobei eine provisorische Zahnprothese auf der Basis des digitalen Modells der Zahnprothese hergestellt wird und diese provisorische Zahnprothese auf dem entsprechenden Kiefer positioniert wird und mittels Tomographie wenigstens ein Bild des Kiefers mit der provisorischen Zahnprothese (1) erzeugt wird, wobei anschließend auf der Basis des Bilds eine Position und eine Ausrichtung für das wenigstens eine Implantat ausgewählt werden,
**dadurch gekennzeichnet, dass** auf der Basis des digitalen Modells der Zahnprothese (1) ein digitales Modell für einen Knochenkammteil (2) erzeugt wird, in dem sich dieser Knochenkammteil gegenüber dem Kamm des Kiefers erstrecken muss und Prothesenzähne enthält, deren Form und gegenseitige Position Prothesenzähnen entsprechen, die auf der Zahnprothese vorgesehen sind, und wobei auf der Basis des digitalen Modells der Zahnprothese auch ein digitales Modell für das Trägerstück (3) erzeugt wird, wobei sich das Trägerstück wenigstens teilweise gegenüber dem Knochen des Kiefers erstrecken sollte, wobei Kopplungsvorrichtungen (6) an dem Trägerstück (3) vorgesehen sind, um den Knochenkammteil (2) auf abnehmbare Weise mit dem Trägerstück (3) zu verbinden, sodass die äußere Form der Gesamtheit des mit dem Trägerstück verbundenen Knochenkammteils im Wesentlichen identisch mit jener der Zahnprothese ist,
wobei der Knochenkammteil (2) und das Trägerstück (3), die auf diese Weise gestaltet wurden, auf der Basis der digitalen Modelle hergestellt werden, wobei basierend auf der ausgewählten Position und Ausrichtung des wenigstens einen Implantats die Bohrschablone (9) derart gestaltet wird, dass diese Bohrschablone an dem Trägerstück (3) befestigt werden kann.

2. Verfahren nach Anspruch 1, wobei eine Einsetzrichtung für das wenigstens eine Implantat ausgehend von dem Bild ausgewählt wird, wobei das digitale Modell für das Trägerstück (3) derart gestaltet wird, dass diese Einsetzrichtung frei bleibt, um entsprechend dieser Einsetzrichtung zu bohren und das Implantat in den Kiefer einzusetzen, wenn dieses Trägerstück passend auf dem Kiefer positioniert ist.

3. Verfahren nach Anspruch 1 oder 2, wobei die digitale Form des Kiefers erzeugt wird, indem eine physische Form des Kiefers gescannt und digitalisiert wird, wobei sich die physische Form virtuell passend mit Weichgewebe des Kiefers verbindet.

4. Verfahren nach Anspruch 3, wobei die physische Form des Kiefers gescannt wird, indem ein optischer Scan, insbesondere ein Laserscan, von wenigstens einem Teil seiner auf das Weichgewebe des Kiefers gerichteten Seite durchgeführt wird.

5. Verfahren nach einem der Ansprüche 3 oder 4, wobei für die physische Form ein bereits vorhandenes herausnehmbares Gebiss verwendet wird.

6. Verfahren nach einem der Ansprüche 1 bis 5, wobei das digitale Modell für das Trägerstück (3) in Abhängigkeit von der Anatomie des betreffenden Kiefers so gestaltet wird, dass sich das Trägerstück (3) wenigstens teilweise vestibulär und/oder palatal zum Knochen des Kiefers erstreckt, wenn es sich um einen Oberkiefer handelt, während, wenn es sich bei dem vorstehend genannten Kiefer um einen Unterkiefer handelt, dieses digitale Modell für das Trägerstück (3) so gestaltet wird, dass sich das Trägerstück (3) vestibulär und/oder lingual zum Knochen eines Unterkiefers erstrecken kann.

7. Verfahren nach einem der Ansprüche 1 bis 6, wobei Montagemittel an dem digitalen Modell der Zahnprothese vorgesehen werden, um es an dem Implantat montieren zu können, nachdem das Implantat im Kiefer fixiert wurde.

8. Verfahren nach einem der Ansprüche 1 bis 7, wobei Befestigungsmittel (12, 13) an dem Trägerstück (3) vorgesehen werden, um es am Knochen des Kiefers fixieren zu können.

9. Verfahren nach einem der Ansprüche 1 bis 8, wobei das mittels Tomographie erzeugte Bild ein dreidimensionales Röntgenbild des Kiefers mit dem Knochenkammteil (2) und dem Trägerstück (3) beinhaltet.

10. Verfahren nach einem der Ansprüche 1 bis 9, wobei der Knochenkammteil (2) und das Trägerstück (3) durch Anwenden einer Rapid-Prototype-Technik, insbesondere durch Fräsen, aus Kunststoff hergestellt werden.

11. Verfahren nach einem der Ansprüche 1 bis 10, wobei die Bohrschablone (9) digital gestaltet wird, wobei sie mittels einer Rapid-Prototype-Technik, insbesondere durch Fräsen, aus Kunststoff hergestellt wird.

12. Zahnprothesensystem mit einer provisorischen Zahnprothese (1) mit Prothesenzähnen für einen Unter- oder Oberkiefer, wobei diese provisorische Zahnprothese passend am Unter- oder Oberkiefer platziert werden kann, wobei sie sich an das Weichgewebe des Kiefers anschmiegt und wobei das Zahnprothesensystem ein Trägerstück (3) und einen Knochenkammteil (2) mit Kopplungsvorrichtungen (6, 7, 8), um das Trägerstück (3) und den Knochenkammteil (2) lösbar miteinander zu verbinden, beinhaltet, wobei sich der Knochenkammteil (2) gegenüber dem Knochenkamm des jeweiligen Kiefers erstrecken sollte und Prothesenzähne enthält, die jenen der provisorischen Zahnprothese entsprechen, während sich das Trägerstück (3) wenigstens teilweise gegenüber dem Knochen dieses Kiefers erstrecken sollte und passend an diesem Kiefer platziert werden kann, wobei eine Ausnehmung (5) in dem Trägerstück (3) vorgesehen ist, die sich gegenüber dem Knochenkamm erstrecken sollte, um das Anbringen von wenigstens einem Implantat in dem Kiefer durch diese Ausnehmung zu ermöglichen, wobei die Gesamtheit aus dem Trägerstück (3) und dem Knochenkammteil (2), nachdem diese mittels der Kopplungsvorrichtungen aneinander befestigt wurden, mittels des Trägerstücks (3) an der gleichen Position wie die provisorische Zahnprothese passend am Kiefer platziert werden kann, wobei die äußere Form der Gesamtheit des mit dem Trägerstück (3) verbundenen Knochenkammteils fast identisch mit jener der Zahnprothese ist, **dadurch gekennzeichnet, dass** sie eine Bohrschablone (9) umfasst, die abnehmbar an dem Trägerstück (3) befestigt werden kann, um diese Bohrschablone (9) gegenüber dem Knochenkamm positionieren zu können, wenn das Trägerstück (3) passend am Kiefer platziert wird, wobei die Bohrschablone (9) wenigstens eine Öffnung (10) zur Führung eines Bohrers aufweist, um ein Bohrloch im Knochen des Kiefers zu bilden und ein Implantat durch die Öffnung (10) in das Bohrloch einzusetzen.

13. Zahnprothesensystem nach Anspruch 12, wobei das Trägerstück (3) dafür eingerichtet ist, sich wenigstens teilweise vestibulär und/oder palatal zum Knochen des Kiefers zu erstrecken, wenn es sich bei dem Kiefer um einen Oberkiefer handelt, während, wenn es sich bei dem Kiefer um einen Unterkiefer handelt, sich das Trägerstück (3) vestibulär und/oder lingual zum Knochen des Unterkiefers erstrecken kann.

14. Zahnprothesensystem nach Anspruch 12 oder 13, wobei das Trägerstück (3) Befestigungsmittel (12, 13) umfasst, die es ermöglichen, es am Kieferknochen zu fixieren.

15. Zahnprothesensystem nach Anspruch 14, wobei die Befestigungsmittel durch Ausnehmungen (12) gebildet sind, die sich zwischen der dem Knochen zugewandten Seite und der vestibulären Seite, der palatalen Seite und/oder der lingualen Seite des Trägerstücks (3) durch das Trägerstück (3) erstrecken, wobei diese Ausnehmungen (12) mit nadelförmigen Verankerungselementen (13) zusammenwirken, die durch diese Ausnehmungen (12) im Kieferknochen zu befestigen sind.

## Revendications

1. Procédé de fabrication d'un gabarit de forage (9) et d'une pièce de support (3) pour positionner ce gabarit de forage (9) par rapport à l'os d'une mâchoire, dans lequel le gabarit de forage (9) comporte au moins une ouverture de guidage (10) permettant de percer des trous dans ledit os en vue d'insérer au moins un implant dans le but de poser une prothèse dentaire (1), dans lequel, selon le procédé, un moule numérique de la mâchoire est généré de telle sorte qu'il puisse être connecté de façon virtuellement ajustée au tissu mou de la mâchoire, dans lequel un modèle numérique pour une prothèse dentaire (1) est conçu sur la base de ce moule numérique de telle sorte qu'il puisse se connecter audit tissu mou de la mâchoire, dans lequel une prothèse dentaire provisoire est fabriquée sur la base dudit modèle numérique de la prothèse dentaire, dans lequel cette prothèse dentaire provisoire est positionnée sur la mâchoire concernée, et au moins une image de la mâchoire munie de la prothèse dentaire provisoire (1) est générée au moyen d'une tomographie, et dans lequel une position et une orientation pour ledit au moins un implant sont alors sélectionnées sur la base de ladite image,
**caractérisé en ce qu'**un modèle numérique pour une partie de crête osseuse (2) est généré sur la base du modèle numérique de la prothèse dentaire (1), dans lequel cette partie de crête osseuse doit s'étendre à l'opposé de la crête de la mâchoire et comprend des dents prothétiques dont la forme et la position mutuelle correspondent aux dents prothétiques prévues sur ladite prothèse dentaire, et dans lequel un modèle numérique pour ladite pièce de support (3) est également généré sur la base du modèle numérique de la prothèse dentaire, dans lequel ladite pièce de support doit s'étendre au moins partiellement à l'opposé de l'os de la mâchoire, dans lequel des dispositifs de couplage (6) sont prévus sur la pièce de support (3) dans le but de connecter de façon détachable la partie de crête osseuse (2) à la pièce de support (3), de telle sorte que la forme extérieure de l'ensemble de la partie de crête osseuse qui est connectée à la pièce de support soit sensiblement identique à celle de ladite prothèse dentaire,
dans lequel la partie de crête osseuse (2) et la pièce de support (3) ainsi conçues sont fabriquées sur la base desdits modèles numériques, et dans lequel ledit gabarit de forage (9) est conçu sur la base de la position et de l'orientation sélectionnées dudit au moins un implant de telle sorte que ce gabarit de forage puisse être attaché à ladite pièce de support (3).

2. Procédé selon la revendication 1, dans lequel une direction d'insertion pour ledit au moins un implant est sélectionnée à partir de ladite image, dans lequel ledit modèle numérique pour ladite pièce de support (3) est conçu de telle sorte que cette direction d'insertion soit laissée libre pour le forage suivant cette direction d'insertion et pour insérer ledit implant dans la mâchoire lorsque cette pièce de support est positionnée de façon ajustée sur la mâchoire.

3. Procédé selon la revendication 1 ou 2, dans lequel ledit moule numérique de la mâchoire est généré par balayage et numérisation d'un moule physique de la mâchoire, ledit moule physique se connectant de façon virtuellement ajustée au tissu mou de la mâchoire.

4. Procédé selon la revendication 3, dans lequel ledit moule physique de la mâchoire est balayé en effectuant un balayage optique, en particulier un balayage laser, d'au moins une partie de son côté qui est orienté en direction du tissu mou de la mâchoire.

5. Procédé selon l'une quelconque des revendications 3 ou 4, dans lequel on utilise pour ledit moule physique une prothèse dentaire amovible déjà disponible.

6. Procédé selon l'une quelconque des revendications 1 à 5, dans lequel ledit modèle numérique pour la pièce de support (3) est conçu, en fonction de l'anatomie de la mâchoire concernée, de telle sorte que la pièce de support (3) s'étende au moins partiellement côté vestibulaire et/ou côté palatin en direction de l'os de la mâchoire s'il s'agit d'une mâchoire supérieure, alors que si la mâchoire susmentionnée est une mâchoire inférieure, ce modèle numérique pour la pièce de support (3) est conçu de telle sorte que la pièce de support (3) puisse s'étendre côté vestibulaire et/ou côté lingual en direction de l'os d'une mâchoire inférieure.

7. Procédé selon l'une quelconque des revendications 1 à 6, dans lequel des moyens de montage sont prévus sur ledit modèle numérique de la prothèse dentaire dans le but de permettre le montage de celui-ci sur ledit implant après que l'implant ait été fixé dans la mâchoire.

8. Procédé selon l'une quelconque des revendications 1 à 7, dans lequel des moyens de fixation (12, 13) sont prévus sur la pièce de support (3) de façon à permettre la fixation de celle-ci sur l'os de la mâchoire.

9. Procédé selon l'une quelconque des revendications 1 à 8, dans lequel ladite image générée au moyen d'une tomographie comprend une image radiologique en trois dimensions de la mâchoire avec ladite partie de crête osseuse (2) et ladite pièce de support (3).

10. Procédé selon l'une quelconque des revendications 1 à 9, dans lequel ladite partie de crête osseuse (2) et ladite pièce de support (3) sont réalisées en plastique en appliquant une technique de prototypage rapide, en particulier par fraisage.

11. Procédé selon l'une quelconque des revendications 1 à 10, dans lequel ledit gabarit de forage (9) est conçu de façon numérique, et dans lequel il est réalisé en plastique en appliquant une technique de prototypage rapide, en particulier par fraisage.

12. Système de prothèse dentaire comprenant une prothèse dentaire provisoire (1) pourvue de dents prothétiques pour une mâchoire inférieure ou supérieure, dans lequel cette prothèse dentaire provisoire peut être placée de façon ajustée sur la mâchoire inférieure ou supérieure, dans lequel elle s'agence contre le tissu mou de la mâchoire et dans lequel le système de prothèse dentaire comprend une pièce de support (3) et une partie de crête osseuse (2) pourvus d'éléments de couplage (6, 7, 8) de manière à pouvoir connecter de façon détachable la pièce de support (3) et la partie de crête osseuse (2) l'une à l'autre, dans lequel la partie de crête osseuse (2) devrait s'étendre à l'opposé de la crête osseuse de la mâchoire respective et comprend des dents prothétiques qui correspondent à celles de la prothèse dentaire provisoire, alors que la pièce de support (3) devrait s'étendre au moins partiellement à l'opposé de l'os de cette mâchoire et peut être placée de façon ajustée sur ladite mâchoire, dans lequel un évidement (5) est prévu dans la pièce de support (3) qui devrait s'étendre à l'opposé de la crête osseuse de manière à permettre la fixation d'au moins un implant dans la mâchoire à travers cet évidement, dans lequel l'ensemble de la pièce de support (3) et de la crête osseuse (2), lorsque celles-ci sont fixées l'une à l'autre par l'intermédiaire desdits dispositifs de couplage, peut être placé de façon ajustée sur la mâchoire à l'aide de la pièce de support (3) dans la même position que ladite prothèse dentaire provisoire, dans lequel la forme extérieure de l'ensemble de la partie de crête osseuse qui est connectée à la pièce de support (3) est pratiquement identique à celle de ladite prothèse dentaire, **caractérisé en ce qu'**il comprend un gabarit de forage (9) qui peut être attaché de façon détachable à la pièce de support (3) en vue de pouvoir positionner ce gabarit de forage (9) à l'opposé de ladite crête osseuse lorsque la pièce de support (3) est placée sur la mâchoire de façon ajustée, dans lequel le gabarit de forage (9) comporte au moins une ouverture (10) servant à guider un foret en vue de pratiquer un trou de forage dans l'os de ladite mâchoire et d'insérer un implant à l'intérieur de ce trou de forage à travers ladite ouverture (10).

13. Système de prothèse dentaire selon la revendication 12, dans lequel ladite pièce de support (3) est conçue de façon à s'étendre au moins partiellement côté vestibulaire et/ou côté palatin en direction de l'os de la mâchoire lorsque la mâchoire est une mâchoire supérieure, alors que lorsque ladite mâchoire est une mâchoire inférieure, la pièce de support (3) peut s'étendre côté vestibulaire et/ou côté lingual en direction de l'os de la mâchoire inférieure.

14. Système de prothèse dentaire selon la revendication 12 ou 13, dans lequel ladite pièce de support (3) comprend des moyens de fixation (12, 13) qui permettent de fixer celle-ci à l'os de la mâchoire.

15. Système de prothèse dentaire selon la revendication 14, dans lequel lesdits moyens de fixation sont formés par des évidements (12) qui s'étendent à travers la pièce de support (3) entre le côté opposé à l'os et le côté vestibulaire, le côté palatin et/ou le côté lingual de la pièce de support (3), dans lequel ces évidements (12) coopèrent avec des éléments d'ancrage en forme d'aiguille (13) qui doivent être attachés à travers ces évidements (12) dans l'os de la mâchoire.
